# EUROPEAN PATENT APPLICATION

(11) **EP 3 608 390 A1**
(43) Date of publication of application: **12.02.2020**
(21) Application number: 18781840.6
(22) Date of filing: 02.04.2018
(51) Int. Cl.: C11C 1/02, A23K 10/20, A23K 20/158, A23L 33/12, A61K 31/201, A61K 35/64, C11B 1/10

(54) **CONJUGATED LINOLEIC ACID COMPOSITION AND METHOD FOR PRODUCING SAME**

(30) Priority: 04.04.2017 JP 2017074328
(71) Applicant: Natori, Syunji, Kitasoma-gun, Ibaraki 300-1622 (JP); Kushima, Mitsutaka, Miyazaki-shi, Miyazaki 880-0916 (JP)
(72) Inventor: NATORI, Shunji, Miyazaki-shi Miyazaki 880-0916 (JP)
(74) Representative: Mewburn Ellis LLP
(86) International application number: PCT/JP2018/014062
(87) International publication number: WO 2018/186327

(57) **Abstract**

The present invention provides a conjugated linoleic acid containing c9,t11-conjugated linoleic acid, wherein the mass ratio of total t10,c12-conjugated linoleic acid to total c9,t11-conjugated linoleic acid is less than 0.25.

## Description

### Technical Field

The present invention relates to a conjugated linoleic acid composition and a method for producing the same.

### Background Art

Conjugated Linoleic Acid (CLA) is a linoleic acid isomer having a partial structure in which two carbon-carbon double bonds are conjugated (in a sequence such as -C=C-C=C-), and is ingested by humans through foods derived from ruminants, for example.

Conjugated linoleic acid is known to have beneficial effects on humans such as a body fat reducing effect, and foods and artificial supplements derived from organisms other than ruminants (for example, chicken meat and eggs) have also been developed.

For example, Patent Literature 1 discloses, for the purpose of constituting an innovative and alternative form of obtaining unsaturated oils and fatty acids, an extract of fly larvae and pupae oil rich in saturated fatty acids, monounsaturated fatty acids and polyunsaturated fatty acids, in which the extract comprises oil extracted from fly (Musca domestica) larvae and pupae comprising saturated fatty acids, monounsaturated fatty acids and polyunsaturated fatty acids, the contents of which are 11 to 16%, 32 to 42% and 25 to 30%, respectively, in the final composition of the oil.

### Citation List

### Patent Literature

Patent Literature 1: Japanese Patent No. 5824450 Non Patent Literature

Non Patent Literature 1: Clement L. et al. Dietary trans-10,cis-12 conjugated linoleic acid induces hyperinsulinemia and fatty liver in the mouse. J. Lipid Res. 43, 1400-1409 (2002)
Non Patent Literature 2: Ip M.M. et al. The t10,c12 isomer of conjugated linoleic acid stimulates mammary tumorigenesis in transgenic mice over-expressing erbB2 in the mammary epithelium. Carcinogenesis 28, 1269-1276 (2007)

### Summary of Invention

### Technical Problem

However, conventional foods derived from ruminants and those derived from organisms other than ruminants have a very low content of conjugated linoleic acid. In addition, although some conventional artificial supplements have a higher content of conjugated linoleic acid than foods derived from ruminant animals, they may also cause, in addition to the beneficial effects on animals, harmful effects on animals such as induction of hyperinsulinemia or fatty liver, carcinogenesis of breast cancer with erbB-2 gene overexpression, promotion of cancer metastasis, and organ enlargement (for example, see Non Patent Literature 1 and Non Patent Literature 2). This is because conjugated linoleic acid has dozens of isomers, and each isomer has a different action on animals.

Therefore, the present invention has been made to solve the above problems of the prior art, and aims to provide conjugated linoleic acid that can be easily produced and contains an isomer of conjugated linoleic acid that produce beneficial effects on animals in a higher content.

### Solution to Problem

As a result of intensive studies to solve the above-described problems of the prior art, the present inventor has found that by using a conjugated linoleic acid containing c9,t11-conjugated linoleic acid and having a mass ratio of t10,c12-conjugated linoleic acid to c9,t11-conjugated linoleic acid of less than 0.25, production can be easily conducted and a content of an isomer of conjugated linoleic acid that produces beneficial effects on animals can be increased. Moreover, the present inventor found that a conjugated linoleic acid derived from specific insects contains a relatively large amount of c9,t11-conjugated linoleic acid and does not contain t10,c12-conjugated linoleic acid, or if contains, only in a very small amount, and completed the present invention.

That is, the present invention is as follows.
[1] A conjugated linoleic acid containing
   c9,t11-conjugated linoleic acid,
   wherein a mass ratio of total t10,c12-conjugated linoleic acid to total c9,t11-conjugated linoleic acid is less than 0.25.
[2] The conjugated linoleic acid according to [1],
   derived from an insect belonging to the family Sarcophagidae.
[3] A conjugated linoleic acid,
   derived from an insect belonging to the family Sarcophagidae.
[4] The conjugated linoleic acid according to [2] or [3],
   wherein the insect is a larva or a pupa of Sarcophaga peregrina.
[5] The conjugated linoleic acid according to any one of [1] to [4],
   wherein the conjugated linoleic acid contains the total c9,t11-conjugated linoleic acid in an amount of more than 80 mass% based on a total amount of total conjugated linoleic acids.
[6] The conjugated linoleic acid according to any one of [1] to [5],
   wherein a content of the total t10,c12-conjugated linoleic acid is less than 20 mass% based on the total amount of the total conjugated linoleic acid.
[7] A glyceride of the conjugated linoleic acid according to any one of [1] to [6].
[8] A food, feed, cosmetic or medicament, containing
   the conjugated linoleic acid according to any one of [1] to [6] or the glyceride according to [7].
[9] A food or medicament used for the treatment or prevention of cancer, obesity, diabetes, arteriosclerosis, immunodeficiency, rheumatoid arthritis, osteoporosis, hypertension or Alzheimer's disease,
   containing the conjugated linoleic acid according to any one of [1] to [6] or the glyceride according to [7] as an active ingredient.
[10] A method for producing a conjugated linoleic acid,
   including a step of extracting conjugated linoleic acid from an insect belonging to the order Diptera.
[11] The method for producing a conjugated linoleic acid according to [10],
   wherein the insect belongs to the family Sarcophagidae.

### Brief Description of Drawing

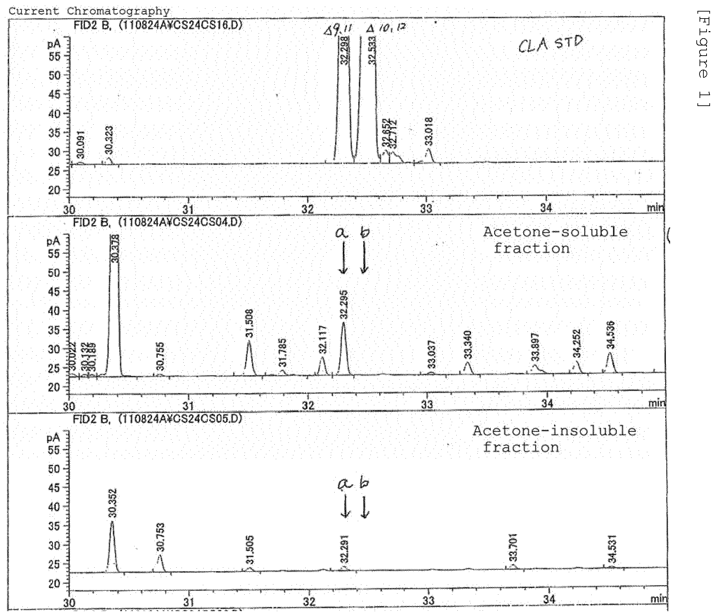

[Figure 1] Figure 1 is the gas chromatography chart measured in the Example.

### Description of Embodiment

Hereinafter, an embodiment of the present invention (hereinafter, referred to as "the present embodiment") will be described in detail. The present embodiment below is an example for explaining the present invention, and is not intended to limit the present invention to the following contents. The present invention can be exploited by appropriately modifying it within the scope of the gist.

### [Conjugated linoleic acid]

The conjugated linoleic acid (hereinafter also simply referred to as "CLA") of the present embodiment contains c9,t11-CLA. The mass ratio of t10,c12-CLA to c9, t11-CLA is less than 0.25. Here, "c", "t", and the following numerical values indicate whether the carbon-carbon double bond is either in a cis- or trans-configuration, and the position of the carbon forming the double bond counting from the carboxylic acid. For example, "c9,t11-CLA" indicates that the carbon-carbon double bonds are in a cis-configuration and a trans-configuration, and formed from carbons at positions 9 and 11, respectively, counting from the carbon of the carboxylic acid.

The CLA of the present embodiment is mainly used to be ingested by animals. That is, using the conjugated linoleic acid of the present embodiment allows to contain c9,t11-CLA, which is a conjugated linoleic acid isomer that produces beneficial effects on animals, in a larger amount and contain t10,c12-CLA in a smaller amount.

The CLA of the present embodiment can be easily produced. This is because CLA having a mass ratio of t10,c12-CLA to c9,t11-CLA of less than 0.25 can be easily obtained, for example, by extraction from insects belonging to the order Diptera, as described later.

The conjugated linoleic acid of the present embodiment means a linoleic acid isomer having a partial structure in which two carbon-carbon double bonds are conjugated (in a sequence such as -C=C-C=C-). CLA is a compound represented by C₁₈H₃₂O₂, and theoretically, 28 isomers exist. Specific examples of CLA isomers include c9,c11-CLA, c9,t11-CLA, t9,c11-CLA, t9,t11-CLA, c10,c12-CLA, c10,t12-CLA, t10,c12-CLA, t10,t12-CLA and t11,t13-CLA.

Since CLA is produced from linoleic acid (n-6) in grass by the action of microorganisms present in the rumen of ruminants, it can be ingested through conventional foods derived from ruminants. However, the amount of CLA in such foods is extremely small, and is about 5 mg per g of fat even in those containing CLA in a large amount.

CLA products are commercially available in Japan and other countries as an anti-obesity supplement that focuses on the effect of reducing body fat of CLA. Commercially available CLA products are not extracted from natural materials, but artificially synthesized by alkaline isomerization of high linoleic acid safflower oil or the like. In conventional CLA products artificially synthesized, the ratio between c9,t11-CLA and t10,c12-CLA is approximately 1: 1.

The physiological effects exhibited by the intake of CLA by animals have been verified mainly in animal experiments, and examples of beneficial physiological effects include a carcinogenesis suppressing effect, a body fat reducing effect, an anti-diabetic effect, an anti-arteriosclerotic effect, an immune enhancing effect, a bone metabolism improving effect, a blood pressure lowering effect, and an anti-rheumatic effect. These beneficial physiological effects are mainly attributed to c9,t11-CLA, and some beneficial physiological effects (for example, body fat reducing effect) are attributed also to t10,c12-CLA.

However, although t10,c12-CLA has some physiological effects in common with the above beneficial physiological effects (for example, a significant body fat reducing effect), it also has harmful physiological effects such as induction of hyperinsulinemia or fatty liver, carcinogenesis of breast cancer with erbB-2 gene overexpression, promotion of cancer metastasis, and organ enlargement, in addition to the beneficial effects on animals. In particular, when women take t10,c12-CLA for a long time as an anti-obesity supplement or the like, an increased risk of developing breast cancer has been pointed out (see Non Patent Literature 2).

The glyceride or sphingoid of the present embodiment is a compound in which CLA is ester-bonded to the hydroxy group of glycerin (for example, triglyceride), or a compound in which CLA is amide-bonded or ester-bonded to the amino group or hydroxy group of sphingosine. Such compounds are obtained as an extract together with free CLA when, for example, CLA is derived from an insect described later. It is also possible to further extract only free CLA or only its triglyceride or sphingoid from the obtained extract using a known method. The glyceride or sphingoid is not particularly limited as long as it has a skeleton forming the above-described ester bond or amide bond, and for example, it may be a compound in which phosphoric acid or sugar is further bonded, or a compound forming a salt.

In the CLA of the present embodiment, the mass ratio of total t10,c12-CLA to total c9,t11-CLA (t10,c12/c9,t11) is less than 0.25, preferably 0.20 or less, more preferably 0.15 or less, further preferably 0.10 or less and furthermore preferably 0.05 or less.

The CLA of the present embodiment contains c9,t11-CLA, and preferably contains the total c9,t11-CLA in an amount of more than 80 mass%, more preferably contains total c9,t11-CLA in an amount of 85 mass% or more, further preferably contains the total c9,t11-CLA in an amount of 90 mass% or more, furthermore preferably contains the total c9,t11-CLA in an amount of 95 mass% or more and furthermore preferably contains the total c9,t11-CLA in an amount of 98 mass% or more, based on the total amount of the total CLA (100 mass%).

The content of the total t10,c12-CLA in the CLA of the present embodiment is preferably less than 20 mass%, more preferably less than 15 mass%, further preferably less than 10 mass% and furthermore preferably less than 5.0 mass%, based on the total amount of the total CLA (100 mass%).

In the present description, the "total c9,t11-CLA" means free c9,t11-CLA, and the triglycerides and sphingoids thereof, all of which are converted into free c9,t11-CLA. In addition, regarding the "total t10,c12-CLA" and the "total CLA", similarly as the "total c9,t11-CLA", each means the free form, and the triglycerides and sphingoids thereof, all of which are converted into the free form.

### [Insects belonging to the order Diptera]

The conjugated linoleic acid (CLA) of the present embodiment may be derived from insects belonging to the order Diptera, in particular from insects belonging to the family Sarcophagidae, Muscidae or Calliphoridae.

Insects belonging to the order Diptera can be easily bred indoors including laboratories. For example, as an insect belonging to the family Sarcophagidae, when Sarcophaga peregrina adults are bred by feeding them powdered milk, sugar and water at 28°C, they mate and lay around 10 days after emergence. The laid larvae can be bred with pork liver and the like, and become third-instar larvae (last instar) in 3 days from the laying, and their volume increases about 350 times from the time of laying. At this point the larvae stop ingestion and hatch. The pupae emerge in about 10 days. In this way, the life cycle of Sarcophaga peregrina is completed in less than one month. Thus, insects belonging to the order Diptera can be bred so that they can easily multiply in large quantities.

In general, animal cells do not have a system for converting linoleic acid (n-6) into CLA, and microorganisms symbiotic or parasitic on the digestive tract of specific animals (for example, ruminants) have this system. In the conventional fatty acid analysis of insects, the presence of CLA has not been reported, and as described later, the CLA contained in Sarcophaga peregrina larvae is a very specific example in terms of its purity and quantity.

CLA derived from insects belonging to the order Diptera has a high purity of c9,t11-CLA as CLA, and its content per lipid is also higher than that from ruminants. Thus, insects belonging to the order Diptera are useful sources of safe natural CLA, c9,t11-CLA.

Insects belonging to the order Diptera are not particularly limited, but examples thereof include insects belonging to the family Fanniidae, Scathophagidae, Anthomyiidae, Sarcophagidae, Muscoidea, Calliphoridae and Drosophilidae, and among these, insects belonging to the family Sarcophagidae are preferable from the viewpoint of reliably achieving the effects of the present invention. Such insects belonging to the family Sarcophagidae include insects belonging to the subfamily Miltogramminae and Sarcophaginae, and these include insects belonging to about 100 genera.

The insects belonging to the Muscoidea family are not particularly limited, and include the housefly Musca domestica.

Specific examples of the insects belonging to the family Sarcophagidae are not particularly limited, and include Amobia distorta, Agria moanchae, Blaeoxipha ampliforceps and Sarcophaga (Boettcherisca) peregrina, and Sarcophaga (Boettcherisca) peregrina is more preferable from the viewpoint of more reliably achieving the effects of the present invention.

Insects belonging to the family Calliphoridae are not particularly limited, and include Lucilia caesar and Lucilia sericata.

It is preferable that the insects belonging to the order Diptera be larvae or pupae thereof. This is because larvae and pupae contain lipids such as CLA rich in calories in a higher amount so that they can induce and maintain metamorphosis until they become adults.

### [Applications]

The CLA and the glycerides and sphingoids thereof of the present embodiment are mainly used to be ingested by animals. In order to realize this, the CLA and the glycerides and sphingoids thereof of the present embodiment can be provided as a food (in particular, functional labeled foods), feed, cosmetics, or medicaments containing the same.

When the CLA or the glyceride or sphingoid thereof of the present embodiment is provided as a food or a medicament containing the same, it can be used for the treatment or prevention of cancer, obesity, diabetes, arteriosclerosis, immunodeficiency, rheumatoid arthritis, osteoporosis, hypertension, or Alzheimer's disease, with CLA as the active ingredient, and among these, it is preferable to use it for the treatment or prevention of cancer or Alzheimer's disease.

Here, regarding to the relationship between conventional CLA and nerve cell protection and brain function improvement related to Alzheimer's disease, it is possible to refer to the following references 1 to 4. The CLA of the present embodiment has a higher purity of c9,t11-CLA compared to conventional CLA, which allows it to more strongly protect nerve cells and improve brain function.
Reference 1: Hunt W.T. et al. Protection of cortical neurons from excitotoxicity by conjugated linoleic acid. J. Neurochem. 115, 123-130 (2010)
Reference 2: Joo N.E. and Park C.S. Inhibition of excitotoxicity in cultured rat cortical neurons by a mixture of conjugated linoleic acid isomers. Pharmacol. Res. 47, 305-310 (2003)
Reference 3: Lee E. et al. Effect of conjugated linoleic acid, µ- calpain inhibitor, on pathogenesis of Alzheimer's disease. Biochim. Biophys. Acta 1831, 709-718 (2003)
Reference 4: Gama M.A.S. et al. Conjugated linoleic acid-enriched butter improved memory and up-regulated phospholipse A2 encoding-genes in rat brain tissue. J. Neural Transm. 122, 1371-1380 (2015)

In the present description, "treatment or prevention" means to produce at least one of cure or remission of the disease, as well as prevention or delay of the onset, prevention or delay of the progression of the disease, alleviation of at least one symptom relating to the disease.

The food, feed, cosmetic, or medicament of the present embodiment contains the CLA of the above present embodiment, and can further contain carriers and additives that are acceptable for food, feed, cosmetics, or medicaments.

Examples of the carriers and the additives include pharmaceutically acceptable organic solvents such as water, saline, phosphate buffer, dextrose, glycerol and ethanol, collagen, polyvinyl alcohol, polyvinyl pyrrolidone, carboxyvinyl polymer, sodium carboxymethyl cellulose, sodium polyacrylate, sodium alginate, watersoluble dextran, sodium carboxymethyl starch, pectin, methyl cellulose, ethyl cellulose, xanthan gum, gum arabic, casein, agar, polyethylene glycol, diglycerin, glycerin, propylene glycol, vaseline, paraffin, stearyl alcohol, stearic acid, human serum albumin, mannitol, sorbitol, lactose and surfactants, but are not limited thereto.

The food, feed, cosmetics, or medicaments of the present embodiment can be provided in the form of a hard capsule, a soft capsule, or a tablet.

### [Method for producing conjugated linoleic acid]

The method for producing a conjugated linoleic acid (CLA) of the present embodiment includes a step of extracting (hereinafter also referred to as "extraction step") a conjugated linoleic acid (also referred to as "extract") from insects belonging to the order Diptera. Moreover, it is preferable that the production method of the present embodiment further includes a step of breeding (hereinafter also referred to as "breeding step") insects belonging to the order Diptera before the extraction step.

### [Breeding step]

In the production method of the present embodiment, larger number and amount of insects belonging to the order Diptera from which CLA is extracted in the extraction step naturally allows to extract a larger amount of CLA. Glycerides and sphingoids of CLA can also be extracted simultaneously with CLA. From such a viewpoint, it is preferable that the production method of the present embodiment includes a breeding step. Including a breeding step allows to obtain a large amount of larvae or pupae belonging to the order Diptera, especially. It is also preferable in terms of hygiene to use newly born larvae in the breeding step. However, the production method of the present embodiment does not need to include a breeding step, and in that case, insects belonging to the order Diptera should be obtained.

As mentioned above, insects belonging to the order Diptera can be easily bred in indoors including laboratories. More specifically, as described in the Examples described later, it is possible to breed insects and obtain a large amount of insect larvae belonging to the order Diptera.

In the breeding step, the feed given to the insects is not particularly limited as long as it is a feed that can be given to insects belonging to the order Diptera, and examples thereof include powdered milk, sugar, pork liver, and safflower oil. Among these, safflower oil is preferable since it tends to increase the CLA contained in insects.

### [Extraction step]

By including an extraction step, the production method of the present embodiment can remove components other than lipids contained in insects, for example components such as proteins, and when the CLA, glycerides or sphingoids obtained by the extraction step are ingested by animals, the effects of the present invention can be reliably achieved.

The insects belonging to the order Diptera are preferably its larvae or pupae. This is because larvae and pupae contain lipids such as CLA rich in calories in a larger amount so that they can induce and maintain metamorphosis until they become adults.

The extraction method is not particularly limited as long as it can extract lipids, and examples thereof include a method of drying and crushing insects and then extracting the lipids with an organic solvent such as a chloroform-methanol mixture. The lipids may include linoleic acid, other saturated fatty acids, and unsaturated fatty acids in addition to CLA.

In addition, the fatty acids extracted with an organic solvent may include fatty acids such as free CLA, CLA glycerides and sphingoids. For example, when extracting with a chloroform-methanol mixture, it is extracted as a mixture of both. Specifically, it can be extracted by the method described in the Example described later.

In addition, when extracting only free fatty acids, it is possible to obtain only free fatty acids by further performing a deesterification treatment (for example, enzyme treatment), and a larger amount of free CLA can be obtained. On the other hand, free CLA can also be converted into glyceride fatty acids by known enzymatic or chemical methods in the presence of glycerol. Therefore, depending on its applications, free CLA, glyceride CLA, or a mixture thereof can each be obtained.

The extraction step allows to obtain CLA with a high purity of c9,t11-CLA.

### Examples

Hereafter, the present invention is further described in detail with Examples, but the present invention is not limited to these Examples. Hereinafter, "%" means mass%, unless otherwise specified.

### [Sarcophaga peregrina breeding]

Sarcophaga peregrina adults were bred in a large-sized breeding cage containing powdered milk, sugar and water, and when they became able to produce offsprings, pork liver slices were inserted into the cage to let them lay larvae on the slices. After the laying, the liver slices were taken out and transferred to a container containing pork liver slices as feed, and the larvae were raised at 28°C. The larvae became 3rd instar larvae after 3 days of growth.

### [Preparation of larva powder]

The third-instar larvae were separated from the feed (the pork liver slices), collected, washed thoroughly with water, and then left for 1 day with their body surface wet to wait for complete digestion of the food in the intestines. Thereafter, the larvae were freeze-dried and powdered to obtain 100 g of larva powder.

### [Lipid extraction]

100 g of the obtained larva powder was extracted with a chloroform-methanol mixture (2:1, v/v) to obtain about 31.8 g of an extracted oil fraction (including CLA and CLA glycerides).

### [CLA detection]

The obtained extracted oil fraction was dissolved in a 0.5 mol/L sodium hydroxide-methanol solution and heated at 100°C for 7 minutes to convert glyceride fatty acids (including CLA glycerides) into free fatty acids. Next, 14% boron trifluoride (BF₃) methanol complex was added to methyl-esterify the fatty acids (including the free CLA), and the fatty acid methyl esters were further purified by TLC (thin layer chromatography). When the purified fatty acid methyl esters were measured by gas chromatography (GC), it was confirmed that 25 fatty acids were contained in the obtained extracted oil fraction: 12:0, 13:0, 14:0, 14:1, 15:0, 16:0, 16:1, 17:0, 18:0, 18:1, 18:2 (including CLA), 18:3, 20:0, 20:1, 20:2, 20:3, 20:5, 22:0, 23:2, 22:6, 23:0, 24:0 and 24:1. Here, the chemical structure of each fatty acid is represented by "X:Y", with X representing the number of carbon atoms and Y representing the degree of unsaturation.

As described below, it was confirmed that the above 18:2 fatty acids included conjugated linoleic acid in addition to normal linoleic acid (n-6). This was identified for the first time with an insect of the order Diptera.

The extracted oil fraction obtained above was treated with acetone, and an acetone-soluble fraction soluble in acetone (fraction of fatty acids and glycerides excluding phospholipids as the main components) and an acetone-insoluble fraction insoluble in acetone (fraction of phospholipids as the main component) was obtained. Next, the acetone-soluble fraction and the acetone-insoluble fraction were each dissolved in a 0.5 mol/L sodium hydroxide-methanol solution and heated at 100°C for 7 minutes to convert glyceride fatty acids (including CLA glycerides) into free fatty acids. Furthermore, 14% boron trifluoride (BF₃) methanol complex was added to each of these acetone-soluble fraction and acetone-insoluble fraction, and c9,t11-CLA and t10,c12-CLA standard products to methyl-esterify the free fatty acids. Thereafter, gas chromatography was further performed on the c9,t11-CLA and t10,c12-CLA standard products methyl-esterified with boron trifluoride, and on the acetone-soluble fraction and acetone-insoluble fraction in which the free fatty acids were methyl-esterified, and the elution positions (retention time) of the GC were compared.

Figure 1 is a gas chromatography chart. The uppermost panel in the figure is a chart obtained by measuring c9,t11-CLA and t10,c12-CLA standard products, and the peaks indicated as "Δ9,11" and "Δ10,12" in the figure correspond to each methyl-esterified standard product. The center panel in the figure is a chart obtained by measuring the methyl-esterified acetone-soluble fraction, the lowermost panel in the figure is a chart obtained by measuring the methyl-esterified acetone-insoluble fraction, and the peaks indicated by "a" and "b" in the figure correspond to the positions where methyl-esterified c9,t11-CLA and t10,c12-CLA were detected, respectively.

As is clear from the results of Figure 1, in both the acetone-soluble fraction and the acetone-insoluble fraction, methyl-esterified c9,t11-CLA was detected, and methyl-esterified t10,c12-CLA and other conjugated linoleic acids were not detected. That is, all (100%) of the detected conjugated linoleic acids derived from the larvae of Sarcophaga peregrina was c9,t11-CLA. Moreover, when a calibration curve was prepared with the methyl-esterified standard products, it was confirmed that 1.58 g of c9,t11-CLA was contained in 100 g of the extracted oil fraction. This value was more than three times the content of c9,t11-CLA in ruminant fat, which is conventionally considered to have the highest content of c9,t11-CLA.

### Industrial Applicability

The conjugated linoleic acid according to the present invention is useful in the fields of food (for example, food for humans and food for pets, in particular functional food), feed (for example, feed for domestic animals), medicaments (for example, medicaments for human and medicaments for pets), cosmetics and the like.

## Claims

1. A conjugated linoleic acid comprising
c9,t11-conjugated linoleic acid,
wherein a mass ratio of total t10,c12-conjugated linoleic acid to total c9,t11-conjugated linoleic acid is less than 0.25.

2. The conjugated linoleic acid according to claim 1,
derived from an insect belonging to the family Sarcophagidae.

3. A conjugated linoleic acid,
derived from an insect belonging to the family Sarcophagidae.

4. The conjugated linoleic acid according to claim 2 or 3,
wherein the insect is a larva or a pupa of Sarcophaga peregrina.

5. The conjugated linoleic acid according to any one of claims 1 to 4,
wherein the conjugated linoleic acid comprises the total c9,t11-conjugated linoleic acid in an amount of more than 80 mass% based on a total amount of the total conjugated linoleic acid.

6. The conjugated linoleic acid according to any one of claims 1 to 5,
wherein a content of the total t10,c12-conjugated linoleic acid is less than 20 mass% based on the total amount of the total conjugated linoleic acid.

7. A glyceride of the conjugated linoleic acid according to any one of claims 1 to 6.

8. A food, feed, cosmetic or medicament, comprising
the conjugated linoleic acid according to any one of claims 1 to 6 or the glyceride according to claim 7.

9. A food or medicament used for the treatment or prevention of cancer, obesity, diabetes, arteriosclerosis, immunodeficiency, rheumatoid arthritis, osteoporosis, hypertension or Alzheimer's disease,
comprising the conjugated linoleic acid according to any one of claims 1 to 6 or the glyceride according to claim 7 as an active ingredient.

10. A method for producing a conjugated linoleic acid,
comprising a step of extracting a conjugated linoleic acid from an insect belonging to the order Diptera.

11. The method for producing a conjugated linoleic acid according to claim 10,
wherein the insect belongs to the family Sarcophagidae.
